# EUROPEAN PATENT SPECIFICATION

(11) **EP 2 332 897 B1**
(45) Date of publication and mention of the grant of the patent: **15.10.2014**
(21) Application number: 08876798.3
(22) Date of filing: 18.08.2008
(51) Int. Cl.: C07C 2/62, C07C 9/14, C10L 1/06, B01J 8/00, C10G 17/02

(54) **METHOD FOR PRODUCING ALKYLBENZENE AND A REACTOR FOR THE SULFURIC ACID ALKYLATION OF ISOBUTANE WITH OLEFINS**
VERFAHREN ZUR HERSTELLUNG VON ALKYLBENZOL UND REAKTOR FÜR DIE SCHWEFELSÄUREALKYLIERUNG VON ISOBUTAN MIT OLEFINEN
PROCÉDÉ DE PRODUCTION D ALKYLATES ET RÉACTEUR D ALKYLATION D ISOBUTANE PAR DES OLÉFINES EN PRÉSENCE D ACIDE SULFURIQUE

(43) Date of publication of application: 15.06.2011
(73) Proprietor: Gershuni, Semen Shikovich, Moscow 119526 (RU); Emelkina, Valentina Andreevna, Moscow 117513 (RU); Zhikharev, Roman Vladimirovich, Moscow 121359 (RU)
(72) Inventor: Gershuni, Semen Shikovich, Moscow 119526 (RU); Emelkina, Valentina Andreevna, Moscow 117513 (RU); Zhikharev, Roman Vladimirovich, Moscow 121359 (RU)
(74) Representative: Kalhammer, Georg
(86) International application number: PCT/RU2008/000558
(87) International publication number: WO 2010/021562

(56) References cited:
- GB-A- 1 172 712
- SU-A1- 977 444
- US-A1- 2003 158 457

## Description

The invention relates to methods and apparatus for obtaining alkylated gasoline by sulphuric acid alkylation of isoparaffins with olefins, principally isobutane with butylene, and can be applied in the oil refining industry.

### Prior art

The most widely used method of producing alkylates (alkylated gasoline) in the oil refining industry involves sulphuric acid alkylation of isobutane with olefins with the object of producing isooctane as the main high-octane constituent of the reaction products.

There are many different known methods of putting into effect the process of sulphuric acid alkylation of isobutane with olefins.

US Patent Nº 3,544,652 of 01.12.70 discloses a method of alkylation of isoparaffin with olefins in the presence of sulphuric acid, wherein the olefin reacts with the alkylating acid-hydrocarbon emulsion formed as a result of thorough mixing of the isoparaffin with the sulphuric acid before contact with the olefin. In this patent the volume ratio of isoparaffin to olefin is preferably equal to 12:1. The volume ratio of acid to hydrocarbons is in the range 2.5:1 to 15:1, but is preferably controlled within 6:1. The reaction takes place adiabatically, preferably in the stream, in a reactor termed an "alkylation contactor" equipped with a mixing device capable of creating an emulsion of isoparaffin with sulphuric acid, and thorough and uniform mixing of the emulsion thus formed with the olefin at the points where it is fed into the reactor.

As the liquid stream moves through the reactor, the temperature of the alkylating mixture continuously increases to 5-15°C, thereby reducing the viscosity of the mixture itself and increasing its degree of turbulence. The process takes place essentially at a temperature between 5 and 60°C and at a pressure necessary to maintain the reagents in liquid phase (between 2 and 10 atm). Before mixing with the isoparaffin the sulphuric acid at a concentration of between 88 and 99% is cooled to temperature of about 4°C.

The method described in US Patent Nº 3,544,652 requires considerable energy inputs for circulating the acid due to its high ratio to the hydrocarbons. This method also requires very large dimensions of the settling equipment. Moreover, in this method the specific consumption of sulphuric acid is high and high quality of the end product is not guaranteed.

A method is known for producing alkylated gasoline by mixing sulphuric acid, isobutane and olefins with a volume ratio of isobutane to olefins between 5:1 and 10:1 [SU Patent Nº 585655, C07C9/16, 1979].

A disadvantage of this method is the low quality of the alkylated gasoline due to the low isobutane-to-olefins ratio in the reaction zone, and also significant losses of sulphuric acid due to the entrainment thereof with the reaction products.

A method is known for producing alkylated gasoline by mixing sulphuric acid with isobutane pre-cooled to a temperature not exceeding minus 2°C, mixing the emulsion obtained with olefins in several stages with cooling to a temperature not exceeding minus 2°C. Mixing of the isobutane with sulphuric acid is accomplished in an injector mixer with an injection coefficient of 3.3 - 5.2; the prepared emulsion is contacted with olefins at a volume ratio of isobutane to olefins of 3000 - 5000 : 1, and separation of the reaction products from the sulphuric acid is carried out using a hydrocyclone [RU Patent Nº 2092475].

In the aforementioned RU Patent Nº 2092475 there is also described an apparatus for producing alkylated gasoline which is a reactor including three injector mixers connected in series. Each mixer is equipped with an inlet device for the olefin feedstock which distributes the feedstock in helical fashion along the length.

The method and apparatus described in RU Patent Nº 2092475 make it possible to put into effect the process of sulphuric acid alkylation of isoparaffins with olefins in a reactor of compact dimensions without moving parts and producing alkylated gasoline of high quality. In practice, however, high energy inputs are required to maintain a ratio of isobutane-to-olefins in the reaction zone in the range 3000 - 5000 : 1.

The sulphuric acid alkylation of isobutane with olefins is also disclosed in US 2003/0158457 and SU 977444.

The method closest to the invention is a method for producing alkylates (alkylated gasoline) by sulphuric acid alkylation of at least one isoparaffin, such as isobutane, with at least one olefin, including (a) preparation of a mixture of the said at least one isoparaffin with the recirculated reaction products by mixing the said at least one isoparaffin pre-cooled to a temperature not exceeding +12°C with the recirculating reaction products separated from the sulphuric acid and also cooled to a temperature not exceeding +12°C; (b) preparation of a first "hydrocarbons in acid" emulsion by injecting the mixture obtained at step (a) in a plurality of parallel streams into a sulphuric acid composition; (c) preparation of a second emulsion by injecting a portion of the said at least one olefin in concurrent streams via nozzles into the first "hydrocarbons in acid" emulsion obtained at step (b). Injection of the second emulsion obtained at step (c) via nozzles into a reaction chamber of specified height and cross-section, wherein the said second emulsion circulates in closed cycle with continuous output of the balance quantity of the reaction mixture; injection of the other portion of the said at least one olefin in concurrent streams via a system of nozzles into the second emulsion circulating in the reaction chamber, wherein said injection is evenly distributed on the cross-section and height of the said reaction chamber; fractionation of the reaction mixture from the reaction chamber using at least one hydrocyclone into an acid-rich fraction and a hydrocarbons-rich fraction, each of which then undergoes pressure reduction and gas separation; recirculation of part of the hydrocarbons-rich phase after vapour separation at step (a), where the said recirculated phase serves as the recirculated reaction products, and output of the remaining part of the hydrocarbons-rich phase for deacidification, purification and fractionation to separate the required alkylate(s) and recirculation of the acid-rich phase after vapour separation and cooling at step (b), where the said circulated acid-rich phase is the said sulphuric acid composition, wherein part of the acid-rich phase is taken off for regeneration before recirculation and replacement by fresh acid [Patent EA 003897].

The nearest equivalent apparatus is an alkylation reactor for producing alkylates, including alkylated gasoline, by sulphuric acid alkylation of at least one isoparaffin such as isobutane, with at least one olefin such as butylene, which includes a mixing chamber for preparing a mixture of the said isoparaffin with circulating reaction products. The apparatus also includes a mixing chamber for preparing a mixture of the said isoparaffin with circulating reaction products, and an emulsion chamber for preparing a first emulsion of hydrocarbons in sulphuric acid, wherein the mixture prepared in the mixing chamber is injected into the sulphuric acid medium in a plurality of parallel streams. The apparatus additionally includes a pre-reaction chamber for preparing a second emulsion, wherein a certain portion of the said olefin is injected in concurrent streams into the first emulsion of hydrocarbons in sulphuric acid fed from the emulsion chamber [Patent EA Nº 003897]. The apparatus also includes a reaction chamber of specified height and transverse cross-section into which the second emulsion from the pre-reaction chamber is injected via nozzles, and the other portion of the olefin is injected in concurrent streams uniformly distributed over the height and cross-section of the reaction chamber. The reaction chamber is arranged so that the second emulsion circulates in a closed loop with the balance quantity of the reaction mixture being continuously drawn off via an outlet connection. The mixing chamber, emulsion chamber, pre-reaction chamber and reaction chamber are mounted coaxially one above other in the vertical position, and together constitute the reactor wherein the pre-reaction chamber is disposed at the bottom of the reactor and the reaction chamber is disposed at the top thereof.

The known method and apparatus make it possible to produce alkylated gasoline with a higher productivity than other known and currently used methods and devices for putting these methods into effect. However, in the course of industrial use of the known invention it was found that in the event of unplanned shutdown of the alkylation reactor the product injection units were prone to acid ingress which, on subsequent restarting of the process, was difficult to remove from low points in the system due to the large density difference between the hydrocarbons and the concentrated sulphuric acid, which had the effect of reducing the operating reliability.

### Disclosure of the invention

The essential purpose of the invention is to propose a method and apparatus for producing alkylated gasoline wherein the operating reliability of the process is increased as well as improving the quality of the alkylated gasoline so produced.

To achieve this purpose, two variants of a method for producing alkylated gasoline are proposed together with two variants of an adiabatic reactor for sulphuric acid alkylation of isobutane with olefins by injection mixing for carrying out the respective proposed variants of the method.

The stated purpose is achieved in that the first variant of the method for producing alkylated gasoline by sulphuric acid alkylation of isobutane with olefins includes the following steps:
a) preparation of a mixture of isobutane with circulating sulphuric acid pre-separated from the reaction products and cooled to a temperature not exceeding +7.0°C;
b) preparation of a mixture of at least part of the stream of olefins-rich feedstock with circulating reaction products pre-separated from the sulphuric acid and cooled to a temperature not exceeding +6.0 C;
c) injection of the mixtures prepared at steps (a) and (b) in separate streams into the circulating loop of a reactor to facilitate the alkylation process;
d) separation of the acid-hydrocarbon emulsion removed from the reactor into two portions, one of which is treated to remove the acid, neutralised and fractionated to separate the isobutane and alkylated gasoline with subsequent return of the isobutane to the alkylation reactor, and the other portion undergoes pressure reduction, gas separation and separation of its liquid part into acid and hydrocarbon components, each of which is returned in separate streams to the alkylation reactor, having first added thereto the circulating isobutane and olefin-rich feedstock in accordance with steps (a) and (b).

Step (a) is accomplished by injecting isobutane into a stream of sulphuric acid via nozzles with a pressure drop at the nozzle not less than 1 kgf/cm² in a special mixer providing uniform distribution of the isobutane in the volume of sulphuric acid. Step (a) is controlled so that the isobutane stream injected into the sulphuric acid amounts to not less than 10% by volume of the sulphuric acid stream.

To put into effect the proposed method for producing alkylated gasoline by sulphuric acid alkylation of isobutane with olefins there is provided a reactor for sulphuric acid alkylation of isobutane with olefins, including a reaction chamber consisting of a vertical cylindrical housing and return pipe coaxial therewith, units for the inlet of sulphuric acid and of circulating reaction products, wherein the sulphuric acid inlet unit is in the form of a chamber with an inlet connection that is situated below the return pipe at a distance from the end thereof and separated therefrom by a transverse partition in which nozzles for introducing the sulphuric acid into the return pipe are secured, said nozzles being oriented upward and being arranged in a circle, the diameter of which is less than the inside diameter of the return pipe, and the unit for the inlet of circulating reaction products mixed with an olefin-containing raw material is in the form of an annular chamber with inlet connection, which chamber is situated above the return pipe and is provided with nozzles that are oriented downwards and are arranged in a circle, the diameter of which is greater than the outside diameter of the return pipe, and with a reaction mixture removal conduit situated inside the annular chamber.

The height of the end clearance between the return pipe and the transverse partition with nozzles situated below it is 0.35 - 0.8 of the outside diameter of the return pipe.

The stated purpose is achieved in that the second variant of the method for producing alkylated gasoline by sulphuric acid alkylation of isobutane with olefins includes the following steps:
a) preparation of a mixture of isobutane with circulating sulphuric acid pre-separated from the reaction products and cooled to a temperature not exceeding +7°C, and injection of this mixture into the lower stage of the alkylation reactor;
b) separation of the stream of olefin-containing feedstock into two portions, one of which is mixed with the circulating reaction products pre-separated from the sulphuric acid and cooled to a temperature not exceeding +6°C, and the other is mixed with part of the stream of circulating isobutane, and injection of these mixtures into the lower and upper stages of the alkylation reactor respectively.
c) removal of the emulsion from the reactor in two streams, one of which is taken off above the level at which the olefin-containing feedstock is introduced into the second stage of the reactor, and the second is taken off below this level, these streams being regulated so that the acid-to-hydrocarbons ratio in both streams differs by no more than 5%.
d) separation of the acid-hydrocarbon emulsion removed from the reactor into two portions, one of which is treated to remove the acid, neutralised and fractionated to separate the isobutane and alkylated gasoline with subsequent return of the isobutane to the alkylation reactor, and the other portion undergoes pressure reduction, gas separation and separation of its liquid part into acid and hydrocarbon components, each of which is returned in separate streams to the alkylation reactor, having first added thereto the circulating isobutane and olefin-rich feedstock in accordance with steps (a) and (b).

Step (a) is accomplished by injecting part of the isobutane stream into a stream of sulphuric acid via nozzles with a pressure drop at the nozzle not less than 1 kgf/cm² in a special mixer providing uniform distribution of the isobutane in the volume of sulphuric acid.

The mixture obtained at step (a) is fed in an ascending stream with a nozzle exit velocity not less than 5m/s into the bottom part of the return pipe of the lower stage of the alkylation reactor, and the mixtures obtained at step (b) are injected in a descending stream into the gap between the return pipe and the housing of each reactor stage.

Part of the isobutane stream is injected into the olefin-rich feedstock stream in the upper stage of the reactor in a quantity necessary to provide a pressure drop at the nozzles not less than 1.5 kgf/cm².

To put into effect the proposed method for producing alkylated gasoline by sulphuric acid alkylation of isobutane with olefins there is provided a two-stage reactor for sulphuric acid alkylation of isobutane with olefins, comprising two vertically coaxial reaction chambers, each of which includes a vertical cylindrical housing and a return pipe coaxial therewith, units for the inlet of olefin-rich feedstock streams in the form of and annular chamber with an inlet connection and nozzles oriented downwards and being arranged in a circle, of which the diameter is greater than the outside diameter of the return pipe, and a reaction mixture removal conduit, the lower chamber is provided with a unit for introducing sulphuric acid mixed with isobutane in the form of a chamber with inlet connection situated below the return pipe and separated therefrom by a transverse partition in which nozzles for introducing sulphuric acid into the mixture with isobutane into the return pipe are secured, said nozzles being oriented upwards and being arranged in a circle, the diameter of which is less than the inside diameter of the return pipe, the conduit for removing the reaction mixture from the lower chamber, situated inside the annular chamber, is separated from the upper chamber by a transverse partition in which nozzles for the flow of emulsion from the lower chamber into the upper chamber are secured, said nozzles being oriented upwards and being arranged in a circle, the diameter of which is less than the inside diameter of the return pipe, there is additionally provided in the housing of the upper chamber above the level of the transverse partition a connection for removal of part of the reaction mixture therefrom.

In each of the reaction chambers the height of the end clearance between the return pipe and the transverse partition with nozzles situated below it is 0.35 - 0.8 of the outside diameter of the return pipe.

The conduit for removing the reaction mixture from the lower chamber is cylindrical in shape with a diameter differing from the inside diameter of the return pipe by no more than 5%.

The annular chamber of the lower reaction chamber is provided with an inlet connection for introducing cooled circulating reaction products mixed with part of the olefin-rich feedstock stream. The annular chamber of the upper reactor stage is provided with an inlet connection for introducing part of the olefin-rich feedstock stream mixed with isobutane.

### Brief description of the drawings

The invention will be more clearly understood by reading the following detailed description of two variants of the reactor for sulphuric acid alkylation of isobutane with olefins and the embodiments of two variants of the proposed method of producing alkylated gasoline according to the invention, with reference to the attached drawings in which:

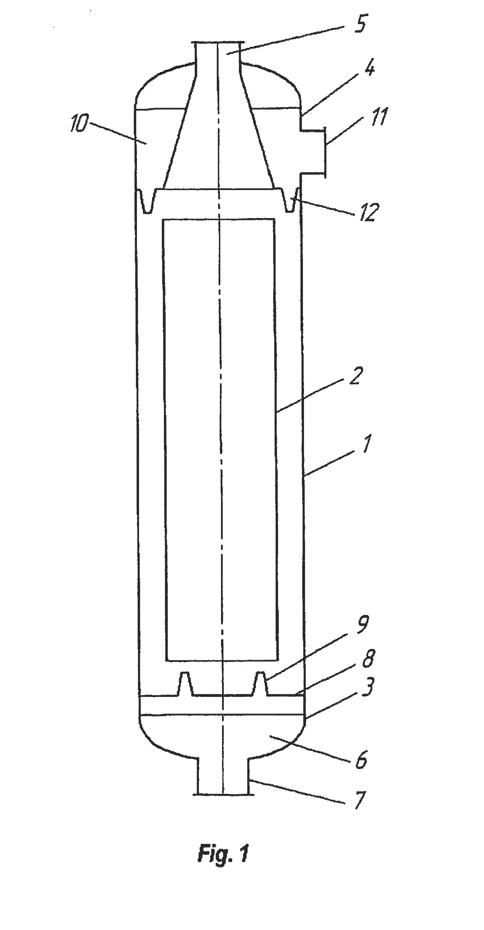

Figure 1 is a schematic representation of a single-stage reactor for sulphuric acid alkylation of isobutane with olefins, comprising the following principal components: a vertical cylindrical housing 1, a return pipe 2 mounted with a gap relative to the housing and coaxial therewith, a unit 3 for introducing sulphuric acid and a unit 4 for introducing circulating reaction products, a conduit 5 for the removal of reaction mixture, mounted inside an annual chamber 10 for the introduction of circulating reaction products. The unit 3 for introducing sulphuric acid is in the form of a chamber 6 with an inlet connection 7 situated below the return pipe 2 with an end clearance relative thereto and separated therefrom by a transverse partition 8, in which nozzles 9 for introducing sulphuric acid into the return pipe 2 are secured, the nozzles being oriented upwards and arranged in a circle, the diameter of which is less than the inside diameter of the return pipe 2. The height of the end clearance between the return pipe and the sulphuric acid inlet chamber is 0.35 - 0.8 of the outside diameter of the return pipe. The unit 4 for introducing circulating reaction products mixed with olefin-rich feedstock is in the form of an annular chamber 10 with an inlet connection 11 situated above the return pipe 2 and provided with nozzles 12 oriented downwards and arranged in a circle, the diameter of which is greater than the outside diameter of the return pipe 2.

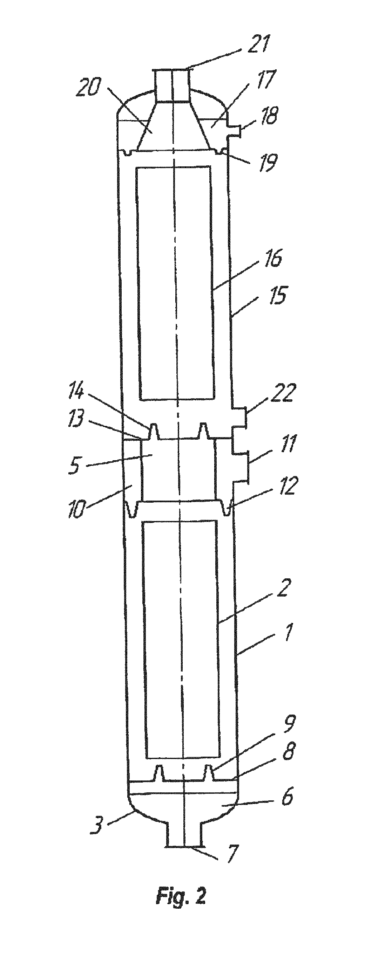

Figure 2 is a schematic representation of a two-stage reactor for sulphuric acid alkylation of isobutane with olefins, comprising two reaction chambers disposed vertically and coaxially one above the other.

The lower chamber of the reactor includes the following components: a vertical cylindrical housing 1, a return pipe 2 mounted at a distance from the housing and coaxially therewith, a unit 3 for introducing sulphuric acid in the form of a chamber 6 with inlet connection 7, situated under the return pipe 2 and separated therefrom by a transverse partition 8, in which nozzles 9 for introducing sulphuric acid are secured; a unit for introducing circulating reaction products mixed with olefin-rich feedstock, in the form of an annular chamber 10 with an inlet connection 11 situated above the return pipe 2 and provided with nozzles 12 oriented downwards; a conduit 5 for removing reaction mixture, mounted inside the annular chamber 10.

In a preferred embodiment of the invention, the conduit 5 is cylindrical in shape with a diameter differing from the inside diameter of the return pipe by no more than 3%.

According to the invention, the conduit 5 of the two-stage reactor is separated from the upper chamber by a transverse partition 13, in which nozzles 14 are secured.

The upper reaction chamber includes a housing 15, a return pipe 16 analogous to the lower return pipe 2, an upper chamber 17 with inlet connection 18 for introducing olefin-rich feedstock, and nozzles 19. The axial cavity of the annular chamber 17 is a conduit 20 for removing reaction mixture, terminating in an upper connection 21. Above the level of the grid 13 there is provided a connection 22 for removing part of the reaction mixture from the upper reaction chamber.

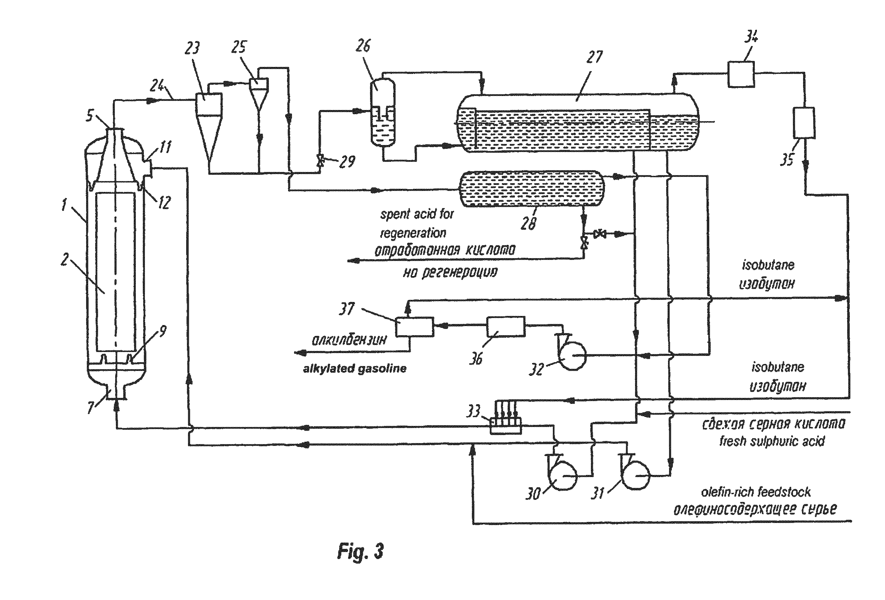

Figure 3 illustrates an embodiment of the first variant of the method for producing alkylated gasoline, comprising a single-stage alkylation reactor 1, a hydrocyclone 23 connected at the inlet by a pipe 24 with the outlet conduit 5 of the reactor, a hydrocyclone 25 connected at the inlet with the upper outlet of the hydrocyclone 23, a gas-liquid separator 26 connected at the inlet with the bottom outlets of the hydrocyclones 23 and 25, a three-phase separator 27, to which are connected the liquid and gas outlets of the separator 26, a liquid settling tank 28 connected at the inlet to the upper outlet of the hydrocyclone 25, a reducer valve 29, circulating pumps 30 and 31, a reaction products transfer pump 32, a mixer 33 for mixing isobutane with sulphuric acid, a compressor 34, a condenser 35, a neutraliser unit 36 and a fractionation unit 37.

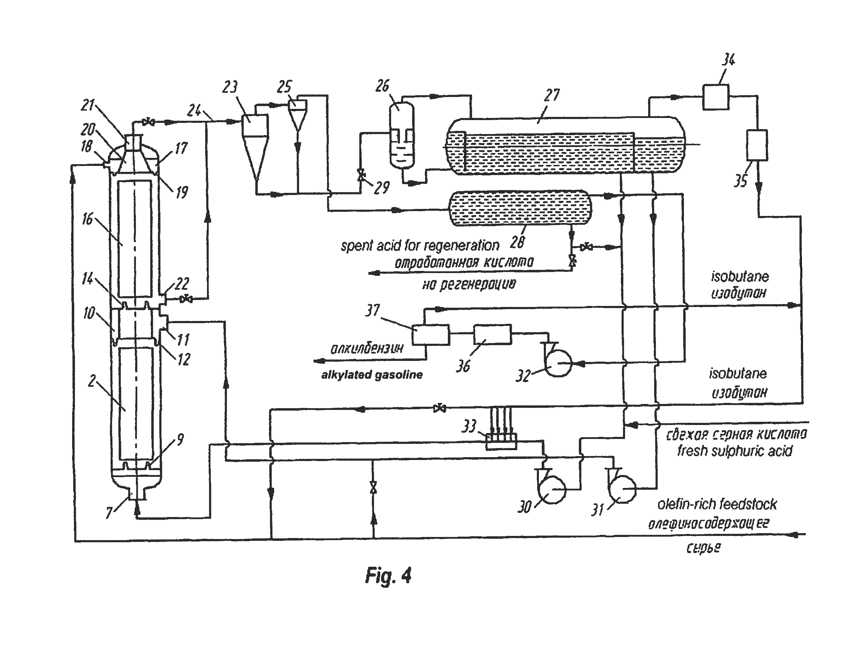

Figure 4 illustrates an embodiment of the second variant of the method for producing alkylated gasoline, comprising a two-stage alkylation reactor as shown in Figure 2, a hydrocyclone 23 connected at the inlet by a pipe 24 with the outlet conduit 20 of the reactor, a hydrocyclone 25 connected at the inlet with the upper outlet of the hydrocyclone 23, a gas-liquid separator 26 connected at the inlet with the bottom outlets of the hydrocyclones 23 and 25, a three-phase separator 27, to which are connected the liquid and gas outlets of the separator 26, a liquid settling tank 28 connected at the inlet to the upper outlet of the hydrocyclone 25, a reducer valve 29, circulating pumps 30 and 31, a reaction products transfer pump 32, a mixer 33 for mixing isobutane with sulphuric acid, a compressor 34, a condenser 35, a neutraliser unit 36 and a fractionation unit 37.

The layout illustrated in Figure 4 differs from that shown in Figure 3 in the following respects:
- part of the olefin-rich feedstock stream is fed to the connection 18 of the annular chamber 17 of the upper reactor stage;
- the line feeding part of the olefin-rich feedstock stream to the upper reactor stage is connected to the isobutane feed line;
- the layout includes two lines for the removal of reaction mixture from the reactor: via the upper connection 21 and via the connection 22 situated above the separating partition 13.

### Detailed description of the invention

The proposed method for producing alkylated gasoline according to the first variant is carried out as follows.

In the mixer 33 there is created a mixture of isobutane with sulphuric acid circulated by means of the pump 30 and pre-separated from the reaction products in the three-phase separator 27 and cooled therein to a temperature not exceeding 7.0°C, and this mixture is then introduced into an alkylation reactor analogous to that shown in Figure 1. The mixture enters the reaction zone via the connection 7 and the nozzles 9.

To the alkylation reaction products, circulated by means of the pump 31 and pre-separated from the sulphuric acid in the three-phase separator 27 and cooled to a temperature not exceeding 6.0°C, is added olefin-rich feedstock and this mixture is then introduced into the alkylation reactor via the connection 11 and the nozzles 12.

In the alkylation reactor, where the pressure is maintained not below 4 kgf/cm² by means of the valve 29, a circulating reaction loop is set up by introducing the products via the nozzles 9 and 12, whereby the stream travels upwards inside the return pipe 2 and travels downwards in the annular gap between the housing 1 and the return pipe 2. There is created in this loop an emulsion of hydrocarbons in sulphuric acid, in which the process of sulphuric acid alkylation of isobutane with olefins takes place.

When the alkylation reaction has taken place, the resulting reaction mixture consisting of an emulsion of hydrocarbons in sulphuric acid is removed from the reactor via the reaction mixture removal conduit 5 situated inside the annular chamber for introducing circulating reaction products 10, and via the pipe 24 into the hydrocyclone 23, where the emulsion is separated out. The light phase of the emulsion is fed from the top of hydrocyclone 23 into hydrocyclone 25.

The acid-hydrocarbon mixture is taken off the bottom of the hydrocyclones 23 and 25, the pressure in this stream being reduced to 0.5 kgf/cm² by means of the valve 29, as a result of which part of the isobutane is evaporated from the said mixture and its temperature is reduced from 8.0-10.0 to 4.5°C. Primary separation of the vapour from the liquid takes place in the gas-liquid separator 26; liquid from the bottom of separator 26 is fed to the bottom of the three-phase separator 27, and vapour from the top of separator 26 is fed to the top of the three-phase separator 27. In the three-phase separator 27 the liquid is divided into circulating acid and hydrocarbons, which are returned to the reactor by means of the pumps 30 and 31 respectively. The isobutane vapour removed by means of the compressor 34 from separator 27 is compressed therein to a pressure of 6 kgf/cm² and condensed in the condenser 35; the liquid isobutane is mixed with isobutane separated from the reaction products in the fractionation unit 37, and returned to the process.

Heat is generated during the alkylation process, therefore the temperature of the stream at the reactor outlet is higher than at the inlet. Cooling of the circulating products takes place by virtue of the evaporation of isobutane during pressure reduction.

The alkylation reactor (Figure 1) works as follows.

Olefin feedstock is introduced into the circulating cooled reaction products. This mixed hydrocarbon stream is introduced via the connection 11 into the annular chamber 10 of the reactor 1. Flowing out through the nozzles 12, the product jets inject the stream ascending via the return pipe 2 of the reactor into the annular gap. The stream of acid introduced into the reactor via the connection 7 of the chamber 6 flows out via the nozzles 9 into this pipe and the emulsion from the annular gap is also injected into the return pipe 2. There is thus created in the reactor an emulsion circulation loop in which the alkylation process takes place.

### Preferred embodiment of the invention

The proposed method for producing alkylated gasoline according to the second variant is carried out as follows.

In the mixer 33 there is created a mixture of isobutane with sulphuric acid circulated by means of the pump 30 and pre-separated from the reaction products in the three-phase separator 27 and cooled therein to a temperature of about 5°C, and this mixture is then introduced into the bottom stage of an alkylation reactor analogous to that shown in Figure 2. The mixture enters the reaction zone via the connection 7 and the nozzles 9.

The stream of olefin-rich feedstock is divided into two parts, one of which is mixed with the alkylation reaction products circulating by means of the pump 31 and pre-separated in the three-phase separator 27 to remove the sulphuric acid and cooled to a temperature not exceeding 7.0°C. This mixture is fed via the connection 11 and the nozzles 12 into the bottom chamber of the alkylation reactor. The other part of the olefin-rich feedstock stream is mixed with part of the circulating isobutane stream and fed via the connection 18 and the nozzles 19 into the upper chamber of the alkylation reactor.

In the reactor, where the pressure is maintained not below 4 kgf/cm² by means of the valve 29, a circulating reaction loop is set up by introducing the products via the nozzles 9 and 12, whereby the stream travels upwards inside the return pipe 2 and travels downwards in the annular gap between the housing 1 and the return pipe 2. There is created in this loop an emulsion of hydrocarbons in sulphuric acid, in which the process of sulphuric acid alkylation of isobutane with olefins takes place.

A second circulation loop is formed in the upper stage of the reactor, where the emulsion emerges via the nozzles 14. An additional quantity of olefin-rich feedstock is fed into this reaction loop.

The reaction mixture, consisting of an emulsion of hydrocarbons in sulphuric acid, is taken off the reactor in two streams via the connections 21 and 22, these streams being regulated so that the ratio of acid to hydrocarbons in both streams differs by no more than 5%. This mixture is fed via the pipe 24 into the hydrocyclone 23, where primary separation of the emulsion takes place, the acid emulsion is drawn off from the bottom of hydrocyclone 23, and the hydrocarbon phase is drawn off from the top and then fed to the hydrocyclone 25.

The hydrocarbon stream, which may contain a small amount of acid, is taken from the top of the hydrocyclone 25 to the settling tank 28, where the residual acid settles out in the bottom of the vessel, from where it is transferred either to the spent acid stream or to the circulating sulphuric acid stream.

The stream of hydrocarbon reaction products from the settling tank 28 is transferred by means of the pump 33 for neutralisation and fractionation with separation of the alkylated gasoline and circulating isobutane.

The acid-hydrocarbon mixture is taken off the bottom of the hydrocyclones 23 and 25, the pressure in this stream being reduced to 0.3 - 0.5 kgf/cm² by means of the valve 29, as a result of which part of the isobutane is evaporated from the said mixture and its temperature is reduced from 8.0-12.0°C to 4-5°C.

The alkylation reactor (Figure 2) works as follows.

The bottom stage of the reactor operates in a manner similar to that of the single-stage reactor shown in Figure 1 and described in variant 1 of the method.

The emulsion flows from the bottom reactor stage via the conduit 5 and the nozzles 14 into the return pipe 16 of the upper reactor stage, thereby injecting emulsion from the annular gap between this pipe 16 and the housing 15. The olefin-rich feedstock introduced via the connection 18 into the upper chamber 17, and which undergoes alkylation, is fed into the descending stream of the circulating loop thus formed via the nozzles 19, thereby making it possible to increase the throughput of the two-stage reactor compared with the single-stage reaction according to variant 1 and to improve the quality of the alkylate by increasing the ratio between isobutane and olefins in the reaction zone.

A high degree of fineness of the injected feedstock is obtained by mixing isobutane with the feedstock stream in a quantity designed to provide a pressure drop at the nozzles 19 not less than 1.5 kgf/cm², and preferably above 2.5 kgf/cm², allowing for the fact that the delivery of feedstock can vary depending on various production factors. When the installation is running at maximum design throughput, the addition of isobutane to the feedstock stream may not be required and all of the isobutane will be introduced into the sulphuric acid stream via the mixer 32.

The emulsion is taken off the reactor in two streams: via the upper connection 21 and via the lower connection 22, the streams being regulated so that the acid-to-hydrocarbon ratio in these streams differs by no more than 5%.

Part of the isobutane stream is injected into the olefin-rich feedstock stream in the upper stage of the reactor used at step (b), in a quantity necessary to provide a pressure drop at the feedstock inlet nozzles to the reactor not less than 1.5 kgf/cm² and preferably above 2.5 kgf/cm².

Compared with the nearest equivalents to the invention, the alkylation reactors represented in Figures 1 and 2 provide greater reliability in operation. This is achieved by placing the hydrocarbon injection nozzles (12 and 19) in the upper part of the reaction chambers and directing the hydrocarbon streams from the top downwards. In this case, even if there is an unplanned shutdown of the reactor or if there is an interruption in the delivery of feedstock, isobutane or circulating reaction products and the annular chambers 10 and 17 fill with acid, when the apparatus is restarted the acid as the heavier denser phase will flow downwards and the annular chamber will refill with hydrocarbons, thereby avoiding the danger of complications (crystallisation and corrosion products in the dead zones of the hydrocarbon inlet units).

Particular examples of the use of the proposed method are presented below.

**Example 1**. Isobutane is fed via the mixer nozzles at a rate of 125 m³/h into the circulating sulphuric acid at a rate of 658 m³/h and this mixture is introduced via the connection 7 and the nozzles 9 into an alkylation reactor analogous to that represented in Figure 1. A mixture of circulating reaction products (274 m³/h) and butane- butylene fraction (isobutane content 48 wt.%, butylenes 46 wt.%) at a rate of 22 m³/h is introduced into the reactor via the connection 11 and the nozzles 12.

Part of the hydrocarbon component is separated from the reaction mixture taken off the reactor in the hydrocyclones unit at the rate of 84 m³/h, which after separation of the residual sulphuric acid in a settling tank is fractionated in a rectification unit yielding 12490 kg/h of alkylated gasoline and 36863 kg/h of isobutane which is returned to the process.

The acid emulsion is removed from the bottom of the hydrocyclones 23 and 25, the pressure in this stream being reduced to 0.5 kgf/cm², as a result of which part of the isobutane evaporates off the emulsion and the temperature is reduced from 7.6 to 4.9°C. In the three-phase separator 27 the liquid is divided into circulating acid and hydrocarbons, which are transferred to the reactor by means of the pumps 30 and 31 respectively. The isobutane vapour taken off the separator 27 by the compressor is compressed to a pressure of 6 kgf/cm², then condensed; the liquid isobutane is mixed with isobutane separated from the reaction products in the fractionation unit and returned to the process.

At the outlet from the fractionation unit the alkylated gasoline had the following characteristics:

| Point | Initial boiling | Boiling | | | End boiling | Octane number | |
|---|---|---|---|---|---|---|---|
| | | 10% | 50% | 90% | | | |
| Temperature °C | 37.2 | 69 | 108 | 161 | 194.5 | RON 97.0 | MON 93.8 |

**Example 2.** Into the bottom reaction chamber of an alkylation reactor analogous to that represented in Figure 2, wherein a pressure not below 4 kgf/cm² is maintained, is introduced via the connection 7 and the nozzles 9 circulating sulphuric acid at the rate of 658 m³/h, and into the reactor via the connection 11 and the nozzles 12 is introduced a mixture of circulating reaction products (274 m³/h, temperature 4.8°C) and butane-butylene fraction (14.5 m³/h, isobutane content 48 wt.%, butylenes 46 wt.%). A mixture of butane-butylene fraction (11.7 m³/h) and isobutane (23.6 m³/h) is introduced into the upper reaction chamber via the connection 18 and the nozzle 19.

The reaction mixture is taken off the reactor via connections 21 (880 m³/h) and 22 (199 m³/h). Part of the hydrocarbon component is then separated from the reaction mixture at the rate of 100.0 m³/h, which after separation of the residual sulphuric acid in the settling tank 28 is fractionated in the rectification unit yielding 14870 kg/h of alkylated gasoline and isobutane which is returned to the process.

Acid-hydrocarbon mixture is taken off the bottom of hydrocyclones 23 and 25 (995 m³/h, including 658 m³/h of acid), the pressure in this stream being reduced to 0.5 kgf/cm2, as a result of which 42470 kg/h of isobutane evaporates off the mixture, and the temperature decreases from 7.8 to 4.9°C. In the three-phase separator 27 the liquid is divided into circulating acid and hydrocarbons, which are returned to the reactor by means of the pumps 30 and 31 respectively. The isobutane vapour taken off the separator 27 by the compressor is compressed to a pressure of 6 kgf/cm², then condensed; the liquid isobutane is mixed with isobutane separated from the reaction products in the fractionation unit and returned to the process.

At the outlet from the fractionation unit the alkylated gasoline had the following characteristics:

| Point | Initial boiling | Boiling | | | End boiling | Octane number | |
|---|---|---|---|---|---|---|---|
| | | 10% | 50% | 90% | | | |
| Temperature °C | 37.2 | 69 | 108 | 162 | 194.5 | RON 97.1 | MON 93.8 |

### Industrial applicability

The proposed invention is intended for the production of alkylated gasoline and makes it possible to increase the operating reliability, production capacity and quality of alkylated gasoline.

## Claims

1. Method for producing alkylated gasoline by sulphuric acid alkylation of isobutane with olefins including the following steps:
a) preparation of a mixture of isobutane with circulating sulphuric acid pre-separated from the reaction products and cooled to a temperature not exceeding +7.0°C;
b) preparation of a mixture of at least part of the stream of olefins-rich feedstock with circulating reaction products pre-separated from the sulphuric acid and cooled to a temperature not exceeding +6.0°C;
c) injection of the mixtures prepared at steps (a) and (b) in separate streams into the circulating loop of a reactor to facilitate the alkylation process;
d) separation of the acid-hydrocarbon emulsion removed from the reactor into two portions, one of which is treated to remove the acid, neutralised and fractionated to separate the isobutane and alkylated gasoline with subsequent return of the isobutane to the alkylation reactor, and the other portion undergoes pressure reduction, gas separation and separation of its liquid part into acid and hydrocarbon components, each of which is returned in separate streams to the alkylation reactor, having first added thereto the circulating isobutane and olefin-rich feedstock in accordance with steps (a) and (b).

2. Method according to claim 1, wherein step (a) is accomplished by injecting isobutane into a stream of sulphuric acid via nozzles with a pressure drop at the nozzle not less than 1 kgf/cm² in a special mixer providing uniform distribution of the isobutane in the volume of sulphuric acid.

3. Method according to claim 2, wherein step (a) is controlled so that the isobutane stream injected into the sulphuric acid amounts to not less than 10% by volume of the sulphuric acid stream.

4. Method for producing alkylated gasoline by sulphuric acid alkylation of isobutane with olefins including the following steps:
a) preparation of a mixture of isobutane with circulating sulphuric acid pre-separated from the reaction products and cooled to a temperature not exceeding +7.0°C, and injection of this mixture into the lower stage of the alkylation reactor;
b) separation of the stream of olefin-containing feedstock into two portions, one of which is mixed with the circulating reaction products pre-separated from the sulphuric acid and cooled to a temperature not exceeding +6.0°C, the other portion being mixed with the circulating isobutane, and injection of these mixtures into the lower and upper stages of the alkylation reactor respectively.
c) removal of the emulsion from the reactor in two streams, one of which is taken off above the level at which the olefin-containing feedstock is introduced into the second stage of the reactor, and the second is taken off below this level, these streams being regulated so that the acid-to-hydrocarbons ratio in both streams differs by no more than 5%.
d) separation of the acid-hydrocarbon emulsion removed from the reactor into two portions, one of which is treated to remove the acid, neutralised and fractionated to separate the isobutane and alkylated gasoline with subsequent return of the isobutane to the alkylation reactor, and the other portion undergoes pressure reduction, gas separation and separation of its liquid part into acid and hydrocarbon components, each of which is returned in separate streams to the alkylation reactor, having first added thereto the circulating isobutane and olefin-rich feedstock in accordance with steps (a) and (b).

5. Method according to claim 4, wherein step (a) is accomplished by injecting isobutane into a stream of sulphuric acid via nozzles with a pressure drop at the nozzle not less than 1 kgf/cm² in a special mixer providing uniform distribution of the isobutane in the volume of sulphuric acid.

6. Method according to claim 4, wherein the mixture obtained at step (a) is fed in an ascending stream with a nozzle exit velocity not less than 5 m/s into the bottom part of the return pipe of the lower stage of the alkylation reactor, and the mixtures obtained at step (b) are injected in a descending stream into the annular gap between the return pipe and the housing of each reactor stage.

7. Method according to claim 4, wherein step (b) is controlled so that part of the flow of olefin-rich feedstock introduced into the upper stage of the alkylation reactor is mixed with a quantity of circulating isobutane such that a pressure drop not less than 1.5 kgf/cm² is provided at the nozzles injecting the resultant mixture into the upper stage of the reactor.

8. Reactor for sulphuric acid alkylation of isobutane with olefins, including a reaction chamber consisting of a vertical cylindrical housing and return pipe coaxial therewith, units for the inlet of sulphuric acid and of circulating reaction products, wherein the sulphuric acid inlet unit is in the form of a chamber with an inlet connection that is situated below the return pipe at a distance from the end thereof and separated therefrom by a transverse partition in which nozzles for introducing the sulphuric acid into the return pipe are secured, said nozzles being oriented upward and being arranged in a circle, the diameter of which is less than the inside diameter of the return pipe, and the unit for the inlet of circulating reaction products mixed with an olefin-containing raw material is in the form of an annular chamber with inlet connection, which chamber is situated above the return pipe and is provided with nozzles that are oriented downwards and are arranged in a circle, the diameter of which is greater than the outside diameter of the return pipe, and with a reaction mixture removal conduit situated inside the annular chamber.

9. Reactor according to claim 8, wherein the height of the end clearance between the return pipe and the transverse partition with nozzles situated below it is 0.35 - 0.8 of the outside diameter of the return pipe.

10. Reactor for sulphuric acid alkylation of isobutane with olefins, comprising two vertically coaxial reaction chambers, each of which includes a vertical cylindrical housing and a return pipe coaxial therewith, a unit for the inlet of olefin-rich feedstock streams in the form of an annular chamber with an inlet connection and nozzles oriented downwards and being arranged in a circle, the diameter of which is greater than the outside diameter of the return pipe, and a reaction mixture removal conduit, the lower chamber is provided with a unit for introducing sulphuric acid mixed with isobutane in the form of a chamber with inlet connection situated below the return pipe and separated therefrom by a transverse partition in which nozzles are secured, said nozzles being oriented upwards and being arranged in a circle, the diameter of which is less than the inside diameter of the return pipe, the conduit for removing the reaction mixture from the lower chamber, situated inside the annular chamber, is separated from the upper chamber by a transverse partition in which nozzles for the flow of emulsion from the lower chamber into the upper chamber are secured, said nozzles being oriented upwards and being arranged in a circle, the diameter of which is less than the inside diameter of the return pipe, there is additionally provided in the housing of the upper chamber above the level of said transverse partition a connection for removal of part of the reaction mixture therefrom.

11. Reactor according to claim 10, wherein the conduit for removing the reaction mixture from the lower reaction chamber is cylindrical in shape with a diameter differing from the inside diameter of the return pipe by no more than 5%.

12. Reactor according to claim 10, wherein the annular chamber of the lower reaction chamber is provided with an inlet connection for introducing cooled circulating reaction products mixed with part of the olefin-rich feedstock stream.

13. Reactor according to claim 10, wherein in each of the reaction chambers the height of the end clearance between the return pipe and the transverse partition with nozzles situated below it is 0.35 - 0.8 of the outside diameter of the return pipe.

14. Reactor according to claim 10, wherein the annular chamber of the upper stage is provided with an inlet connection for introducing part of the olefin-rich feedstock stream mixed with isobutane.

## Patentansprüche

1. Verfahren zur Erzeugung von alkyliertem Benzin durch Schwefelsäurealkylierung von Isobutan mit Olefinen, umfassend die folgenden Schritte:
a) Herstellen eines Gemisches aus Isobutan mit im Kreislauf geführter Schwefelsäure, die im Voraus von den Reaktionsprodukten abgetrennt und auf eine Temperatur, die +7,0 °C nicht übersteigt, abgekühlt wurde;
b) Herstellen eines Gemisches aus zumindest einem Teil des Stroms eines olefinreichen Einsatzstoffes mit im Kreislauf geführten Reaktionsprodukten, die im Voraus von der Schwefelsäure abgetrennt und auf eine Temperatur, die +6,0 °C nicht übersteigt, abgekühlt wurden;
c) Einspritzen der in den Schritten (a) und (b) hergestellten Gemische in separaten Strömen in die Kreislaufschleife eines Reaktors zur Erleichterung des Alkylierungsprozesses;
d) Trennen der aus dem Reaktor entfernten Säure-Kohlenwasserstoff-Emulsion in zwei Teile, von denen einer zur Entfernung der Säure behandelt, neutralisiert und fraktioniert wird, um das Isobutan und das alkylierte Benzin zu trennen, wobei das Isobutan anschließend in den Alkylierungsreaktor zurückgeführt wird, und der andere Teil der Druckminderung, Gastrennung und Trennung seines flüssigen Teils in Säure- und Kohlenwasserstoffkomponenten unterzogen wird, wobei beide in separaten Strömen in den Alkylierungsreaktor zurückgeführt werden, dem zunächst das im Kreislauf geführte Isobutan und der olefinreiche Einsatzstoff gemäß den Schritten (a) und (b) zugegeben worden sind.

2. Verfahren nach Anspruch 1, wobei Schritt (a) ausgeführt wird durch Einspritzen von Isobutan in einen Strom aus Schwefelsäure über Düsen mit einem Druckabfall an der Düse von nicht weniger als 1 kgf/cm² in einem speziellen Mischer, der für eine gleichmäßige Verteilung des Isobutans in dem Volumen an Schwefelsäure sorgt.

3. Verfahren nach Anspruch 2, wobei Schritt (a) so gesteuert wird, dass der in die Schwefelsäure eingespritzte Isobutanstrom nicht weniger als 10 Volumen-% des Schwefelsäurestroms ausmacht.

4. Verfahren zur Erzeugung von alkyliertem Benzin durch Schwefelsäurealkylierung von Isobutan mit Olefinen, umfassend die folgenden Schritte:
a) Herstellen eines Gemisches aus Isobutan mit im Kreislauf geführter Schwefelsäure, die im Voraus von den Reaktionsprodukten getrennt und auf eine Temperatur, die +7,0 °C nicht übersteigt, abgekühlt wurde, und Einspritzen dieses Gemisches in die untere Stufe des Alkylierungsreaktors;
b) Trennen des Stroms von Olefin-enthaltendem Einsatzstoff in zwei Teile, von denen einer mit den im Kreislauf geführten Reaktionsprodukten gemischt wird, die im Voraus von der Schwefelsäure getrennt und auf eine Temperatur, die +6,0 °C nicht übersteigt, abgekühlt wurden, und der andere Teil mit dem im Kreislauf geführten Isobutan gemischt wird, und Einspritzen dieser Gemische in die untere bzw. die obere Stufe des Alkylierungsreaktors;
c) Entfernen der Emulsion aus dem Reaktor in zwei Strömen, von denen einer über dem Pegel, bei dem der Olefin-enthaltende Einsatzstoff in die zweite Stufe des Reaktors eingeführt wird, entnommen wird und der zweite unter diesem Pegel entnommen wird, wobei diese Ströme so reguliert werden, dass sich das Verhältnis von Säure zu Kohlenwasserstoffen in beiden Strömen um nicht mehr als 5 % unterscheidet;
d) Trennen der aus dem Reaktor entfernten Säure-Kohlenwasserstoff-Emulsion in zwei Teile, von denen der eine zur Entfernung der Säure behandelt, neutralisiert und fraktioniert wird, um das Isobutan und das alkylierte Benzin zu trennen, wobei das Isobutan anschließend in den Alkylierungsreaktor zurückgeführt wird, und der andere Teil der Druckminderung, Gastrennung und Trennung seines flüssigen Teils in Säure- und Kohlenwasserstoffkomponenten unterzogen wird, wobei beide in separaten Strömen in den Alkylierungsreaktor zurückgeführt werden, dem zunächst das im Kreislauf geführte Isobutan und der olefinreiche Einsatzstoff gemäß den Schritten (a) und (b) zugegeben worden sind.

5. Verfahren nach Anspruch 4, wobei Schritt (a) ausgeführt wird durch Einspritzen von Isobutan in einen Strom von Schwefelsäure über Düsen mit einem Druckabfall an der Düse von nicht weniger als 1 kgf/cm² in einem speziellen Mischer, der für eine gleichmäßige Verteilung des Isobutans in dem Volumen an Schwefelsäure sorgt.

6. Verfahren nach Anspruch 4, wobei das in Schritt (a) erhaltene Gemisch in einem aufsteigenden Strom mit einer Düsenaustrittsgeschwindigkeit von nicht weniger als 5 m/s in den unteren Teil des Rücklaufrohrs der unteren Stufe des Alkylierungsreaktors gespeist wird und die in Schritt (b) erhaltenen Gemische in einem absteigenden Strom in den ringförmigen Spalt zwischen dem Rücklaufrohr und dem Gehäuse jeder Reaktorstufe eingespritzt werden.

7. Verfahren nach Anspruch 4, wobei Schritt (b) so gesteuert wird, dass ein Teil des in die obere Stufe des Alkylierungsreaktors eingeführten Stroms des olefinreichen Einsatzstoffes mit einer Menge an im Kreislauf geführtem Isobutan derart gemischt wird, dass ein Druckabfall von nicht weniger als 1,5 kgf/cm² an den Düsen vorherrscht, die das resultierende Gemisch in die obere Stufe des Reaktors einspritzen.

8. Reaktor zur Schwefelsäurealkylierung von Isobutan mit Olefinen, umfassend eine Reaktionskammer, bestehend aus einem vertikalen zylindrischen Gehäuse und einem damit koaxialen Rücklaufrohr, Einheiten zum Einlass von Schwefelsäure und im Kreislauf geführten Reaktionsprodukten, wobei die Schwefelsäure-Einlasseinheit in Form einer Kammer mit einer Einlassverbindung vorliegt, die sich unter dem Rücklaufrohr in einem Abstand von dessen Ende und getrennt davon durch eine Quertrennwand befindet, in der Düsen zum Einführen der Schwefelsäure in das Rücklaufrohr befestigt sind, wobei die Düsen nach oben ausgerichtet und in einem Kreis angeordnet sind, dessen Durchmesser kleiner ist als der Innendurchmesser des Rücklaufrohrs, und die Einheit zum Einlass der im Kreislauf geführten Reaktionsprodukte, die mit einem Olefin-enthaltenden Rohmaterial gemischt wurden, in Form einer ringförmigen Kammer mit Einlassverbindung vorliegt, wobei sich die Kammer über dem Rücklaufrohr befindet und mit Düsen versehen ist, die nach unten ausgerichtet und in einem Kreis angeordnet sind, dessen Durchmesser größer ist als der Außendurchmesser des Rücklaufrohrs, und wobei sich eine Leitung zur Entfernung des Reaktionsgemisches in der ringförmigen Kammer befindet.

9. Reaktor nach Anspruch 8, wobei die Höhe des freien Raums am Ende zwischen dem Rücklaufrohr und der Quertrennwand, worunter sich Düsen befinden, 0,35 - 0,8 des Außendurchmessers des Rücklaufrohrs beträgt.

10. Reaktor zur Schwefelsäurealkylierung von Isobutan mit Olefinen, umfassend zwei vertikal koaxiale Reaktionskammern, die jeweils ein vertikales zylindrisches Gehäuse und ein damit koaxiales Rücklaufrohr umfassen, eine Einheit zum Einlass von Strömen eines olefinreichen Einsatzstoffes in Form einer ringförmigen Kammer mit einer Einlassverbindung und Düsen, die nach unten ausgerichtet und in einem Kreis angeordnet sind, dessen Durchmesser größer ist als der Außendurchmesser des Rücklaufrohrs, und eine Leitung zum Entfernen des Reaktionsgemisches, wobei die untere Kammer mit einer Einheit zum Einführen von mit Isobutan gemischter Schwefelsäure in Form einer Kammer mit Einlassverbindung versehen ist, die sich unter dem Rücklaufrohr und davon getrennt durch eine Quertrennwand befindet, in der Düsen befestigt sind, wobei die Düsen nach oben ausgerichtet und in einem Kreis angeordnet sind, dessen Durchmesser kleiner ist als der Innendurchmesser des Rücklaufrohrs, wobei die Leitung zum Entfernen des Reaktionsgemisches aus der unteren Kammer, die sich in der ringförmigen Kammer befindet, von der oberen Kammer durch eine Quertrennwand getrennt ist, in der Düsen für den Fluss der Emulsion aus der unteren Kammer in die obere Kammer befestigt sind, wobei die Düsen nach oben ausgerichtet und in einem Kreis angeordnet sind, dessen Durchmesser kleiner ist als der Innendurchmesser des Rücklaufrohrs, wobei zusätzlich in dem Gehäuse der oberen Kammer über der Höhe der Quertrennwand eine Verbindung zum Entfernen eines Teils des Reaktionsgemisches daraus vorgesehen ist.

11. Reaktor nach Anspruch 10, wobei die Leitung zum Entfernen des Reaktionsgemisches aus der unteren Reaktionskammer zylindrisch mit einem Durchmesser, der sich vom Innendurchmesser des Rücklaufrohrs um nicht mehr als 5 % unterscheidet, ist.

12. Reaktor nach Anspruch 10, wobei die ringförmige Kammer der unteren Reaktionskammer mit einer Einlassverbindung zum Einführen gekühlter im Kreislauf geführter Reaktionsprodukte, die mit einem Teil des Stroms von olefinreichem Einsatzstoff gemischt sind, versehen ist.

13. Reaktor nach Anspruch 10, wobei in jeder Reaktionskammer die Höhe des freien Raums am Ende zwischen dem Rücklaufrohr und der Quertrennwand, worunter sich Düsen befinden, 0,35 - 0,8 des Außendurchmessers des Rücklaufrohrs beträgt.

14. Reaktor nach Anspruch 10, wobei die ringförmige Kammer der oberen Stufe mit einer Einlassverbindung zum Einführen eines Teils des mit Isobutan gemischten Stroms von olefinreichem Einsatzstoff versehen ist.

## Revendications

1. Procédé de production d'essence alkylée par alkylation à l'acide sulfurique d'isobutane avec des oléfines comprenant les étapes suivantes :
a) préparation d'un mélange d'isobutane avec de l'acide sulfurique circulant préalablement séparé des produits de réaction et refroidit à une température ne dépassant pas +7,0 °C ;
b) préparation d'un mélange d'au moins une partie d'un courant de charge d'alimentation riche en oléfines avec les produits de réaction en circulation préalablement séparés de l'acide sulfurique et refroidis à une température ne dépassant pas +6,0 °C ;
c) injection des mélanges préparés aux étapes (a) et (b) en des courants séparés dans la boucle de circulation d'un réacteur pour faciliter le procédé d'alkylation ;
d) séparation en deux portions de l'émulsion d'acide hydrocarboné évacuée du réacteur, dont l'une est traitée pour évacuer l'acide, neutralisée et fractionnée, pour séparer l'isobutane et l'essence alkylée avec le retour ultérieur de l'isobutane dans le réacteur d'alkylation, et l'autre portion subit une réduction de pression, la séparation de gaz et la séparation de sa portion liquide dans les composants acides et d'hydrocarbures, chacun d'entre eux étant renvoyé dans des courants séparés vers le réacteur d'alkylation, après avoir d'abord ajouté à celui-ci l'isobutane en circulation et la charge d'alimentation riche en oléfines conformément aux étapes (a) et (b).

2. Procédé selon la revendication 1, dans lequel l'étape (a) est réalisée en injectant de l'isobutane dans un courant d'acide sulfurique par l'intermédiaire de buses avec une chute de pression à la buse pas inférieure à 1 kgf/cm² dans un mélangeur spécial fournissant une distribution uniforme de l'isobutane dans le volume d'acide sulfurique.

3. Procédé selon la revendication 2, dans lequel l'étape (a) est commandée de telle sorte que le courant d'isobutane injecté dans l'acide sulfurique devient pas inférieure à 10 % en volume du courant d'acide sulfurique.

4. Procédé de production d'essence alkylée par alkylation à l'acide sulfurique d'isobutane avec des oléfines comprenant les étapes suivantes :
a) préparation d'un mélange d'isobutane avec de l'acide sulfurique circulant préalablement séparé des produits de réaction et refroidit à une température ne dépassant pas +7,0 °C, et l'injection de ce mélange dans l'étage inférieure du réacteur d'alkylation ;
b) séparation du courant de charge d'alimentation contenant des oléfines en deux portions, dont l'une est mélangée avec les produits de réaction circulant préalablement séparés de l'acide sulfurique et refroidis jusqu'à une température ne dépassant pas +6.0 °C, l'autre portion étant mélangée avec l'isobutane en circulation, et l'injection de ces mélanges dans les étages inférieurs et supérieurs du réacteur d'alkylation respectivement ;
c) évacuation de l'émulsion à partir du réacteur en deux courants, dont l'un est soutiré au-dessus du niveau auquel la charge d'alimentation contenant des oléfines est introduite dans le second étage du réacteur, et dont le second est soutiré en-dessous de ce niveau, ces courants étant régulés de telle sorte que le rapport entre l'acide et les hydrocarbures dans les deux courants ne diffère pas de plus de 5 % ;
d) séparation de l'émulsion acide hydrocarbonée évacuée du réacteur en deux portions, dont l'une est traitée pour évacuer l'acide, neutralisé et fractionné, pour séparer l'isobutane et l'essence alkylée avec le retour ultérieur de l'isobutane dans le réacteur d'alkylation, et l'autre portion subit une réduction de pression, la séparation de gaz et la séparation de la partie liquide dans les composants acides et d'hydrocarbures, chacun d'entre eux étant renvoyé dans des courants séparés vers le réacteur d'alkylation, après avoir d'abord ajouté à celui-ci l'isobutane en circulation et la charge d'alimentation riche en oléfines conformément aux étapes (a) et (b).

5. Procédé selon la revendication 4, dans lequel l'étape (a) est réalisée en injectant de l'isobutane dans un courant d'acide sulfurique par l'intermédiaire de buses avec une chute de pression à la buse pas inférieure à 1 kgf/cm² dans un mélangeur spécial fournissant une distribution uniforme de l'isobutane dans le volume d'acide sulfurique.

6. Procédé selon la revendication 4, dans lequel le mélange obtenu à l'étape (a) est alimenté dans un courant ascendant avec une vitesse de sortie de la buse qui n'est pas inférieure à 5 m/s dans la partie de fond de la conduite de retour de l'étage inférieur du réacteur d'alkylation, et les mélanges obtenus à l'étape (b) sont injectés dans un courant descendant dans l'espace annulaire entre la conduite de retour et le boîtier de chaque étage de réacteur.

7. Procédé selon la revendication 4, dans lequel l'étape (b) est contrôlée de sorte qu'une partie de l'écoulement de la charge d'alimentation riche en oléfines introduite dans l'étage supérieur du réacteur d'alkylation est mélangée avec une quantité d'isobutane en circulation de telle sorte qu'une chute de pression pas inférieure à 1,5 kgf/cm² est prévue au niveau des buses injectant le mélange résultant dans l'étage supérieure du réacteur.

8. Réacteur d'alkylation d'acide sulfurique de l'isobutane par des oléfines, y compris une chambre de réaction constituée d'un boîtier cylindrique vertical et d'une conduite de retour coaxiale, unités pour l'accès de l'acide sulfurique et pour faire circuler les produits de réaction, dans lequel l'unité d'accès de l'acide sulfurique présente la forme d'une chambre avec un raccord d'accès qui est situé en-dessous de la conduite de retour à distance de l'extrémité de celui-ci et séparé de celui-ci par une cloison transversale dans laquelle les buses pour l'introduction de l'acide sulfurique dans la conduite de retour sont fixées, lesdites buses étant orientées vers le haut et étant disposées dans un cercle, dont le diamètre est inférieur au diamètre intérieur de la conduite de retour, et l'unité pour l'accès de produits de réaction circulants mélangés avec une matière première contenant des oléfines est sous la forme d'une chambre annulaire avec le raccord d'accès, laquelle chambre est située au-dessus de la conduite de retour et est pourvue de buses qui sont orientées vers le bas et qui sont disposées en cercle, dont le diamètre est supérieur au diamètre extérieur de la conduite de retour, et avec une conduite d'évacuation du mélange de réaction située à l'intérieur de la chambre annulaire.

9. Réacteur selon la revendication 8, dans lequel la hauteur de l'espace libre entre la conduite de retour et la cloison transversale avec les buses situées au-dessous est 0,35 - 0,8 du diamètre extérieur de la conduite de retour.

10. Réacteur d'alkylation d'acide sulfurique de l'isobutane par des oléfines, comprenant deux chambres de réaction verticalement coaxiales, dont chacune comprend un boîtier cylindrique vertical et une conduite de retour coaxiale, une unité pour l'accès du courant de charges d'alimentation riches en oléfines présentant la forme d'une chambre annulaire avec un raccord d'accès et de buses orientées vers le bas et étant disposées en un cercle, le diamètre duquel est plus grand que le diamètre extérieur de la conduite de retour, et une conduite d'évacuation de mélange de réaction, la chambre inférieure est munie d'une unité pour l'accès d'acide sulfurique mélangé avec de l'isobutane présentant la forme d'une chambre avec une connexion d'accès située au-dessous de la conduite de retour et séparée de ce dernier par une cloison transversale dans laquelle les buses sont fixées, lesdites buses étant orientées vers le haut et étant disposées en un cercle, dont le diamètre est inférieur au diamètre intérieur de la conduite de retour, la conduite pour évacuer le mélange de réaction à partir de la chambre inférieure, située à l'intérieur de la chambre annulaire, est séparée de la chambre supérieure par une cloison transversale dans laquelle les buses pour l'écoulement de l'émulsion de la chambre inférieure à la chambre supérieure sont fixées, lesdites buses étant orientées vers le haut et étant disposées en un cercle, dont le diamètre est inférieur au diamètre intérieur de la conduite de retour, il est en outre prévu dans le boîtier de la chambre supérieure au-dessus du niveau de ladite cloison transversale un raccord pour évacuer une partie du mélange de réaction de celui-ci.

11. Réacteur selon la revendication 10, dans lequel la conduite pour évacuer le mélange de réaction à partir de la chambre de réaction inférieure est de forme cylindrique avec un diamètre différent du diamètre intérieur de la conduite de retour de pas plus de 5 %.

12. Réacteur selon la revendication 10, dans lequel la chambre annulaire de la chambre de réaction inférieure est munie d'un raccord d'accès pour l'introduction de produits de réaction circulant refroidi mélangés avec une partie du courant de charge d'alimentation riche en oléfines.

13. Réacteur selon la revendication 10, dans lequel dans chacune des chambres de réaction la hauteur du dégagement entre la conduite de retour et la cloison transversale avec les buses situées au-dessous, il est de 0,35 à 0,8 du diamètre extérieur de la conduite de retour.

14. Réacteur selon la revendication 10, dans lequel la chambre annulaire de l'étage supérieur est pourvue d'un raccord d'accès pour l'introduction d'une partie du courant de charge d'alimentation riche en oléfines mélangé avec de l'isobutane.
